# EUROPÄISCHE PATENTSCHRIFT

(11) **EP 1 304 318 B1**
(45) Veröffentlichungstag und Bekanntmachung des Hinweises auf die Patenterteilung: **11.01.2006**
(21) Anmeldenummer: 02021132.2
(22) Anmeldetag: 23.09.2002
(51) Int. Cl.: C07C 5/03, C07C 5/08, C07C 9/02, C07C 9/14, C07C 5/09, C07C 11/04, C07C 11/06, C07B 35/02

(54) **Verfahren zur selektiven katalytischen Gasphasenhydrierung von Alkinen, Dienen, Alkeninen und/oder Polyenen**
Process for selective catalytic vapour phase hydrogenation of alkines, dienes, alkenines and/or polyenes
Procédé d'hydrogénation catalytique sélective en phase gazeuse d'alkines, de diènes, d'alkénines et/ou de polyènes

(30) Priorität: 09.10.2001 DE 10149631
(43) Veröffentlichungstag der Anmeldung: 23.04.2003
(73) Patentinhaber: BASF Aktiengesellschaft, 67056 Ludwigshafen (DE)
(72) Erfinder: Hill, Thomas, Dr., 68159 Mannheim (DE); Haake, Mathias, Dr., 68161 Mannheim (DE); Schwab, Ekkehard, Dr., 67434 Neustadt (DE); Frenzel, Andrea, Dr., 68535 Edingen-Neckarhausen (DE); Wörz, Helmut, Dr., 68219 Mannheim (DE)

(56) Entgegenhaltungen:
- EP-A- 0 087 980
- DE-A- 2 854 698

## Beschreibung

Die vorliegende Erfindung betrifft ein Verfahren zur selektiven katalytischen Gasphasenhydrierung von Alkinen, Dienen, Alkeninen und/oder Polyenen in einem Olefin enthaltenden Kohlenwasserstoffstrom.

In Raffinerien und petrochemischen Anlagen werden in großem Umfang Kohlenwasserstoffströme erzeugt, gelagert und verarbeitet. In diesen Kohlenwasserstoffströmen sind häufig hochungesättigte Verbindungen vorhanden, deren Anwesenheit insbesondere bei Verarbeitung und/oder Lagerung bekanntermaßen zu Problemen führt, oder die nicht das gewünschte Wertprodukt darstellen und die daher unerwünschte Komponenten der entsprechenden Kohlenwasserstoffströme sind. Diese hochungesättigten Verbindungen sind Alkine, Diene, Alkenine und/oder Polyene, die in aller Regel höher ungesättigte Homologe des im betreffenden Kohlenwasserstoffstrom enthaltenen Wertprodukts sind, das meist ein einfach ungesättigtes Olefin oder auch 1,3-Butadien ist. Beispielsweise ist in C₂-Strömen von Steamcrackern die Nebenkomponente Ethin (Trivialname "Acetylen") unerwünscht und Ethen (Trivialname "Ethylen") ist das Wertprodukt, in C₃-Strömen sind die Nebenkomponenten Propin und Propadien (Trivialnamen "Methylacetylen" und "Allen", respektive) unerwünscht und Propen (Trivialname "Propylen") ist das Wertprodukt, und in C₄-Strömen sind die Nebenkomponenten 1-Butin, 2-Butin, But-3-en-1-in (Trivialname "Vinylacetylen"), 1,2-Butadien und Butatrien unerwünscht, wenn 1,3-Butadien als Wertprodukt gewonnen und weiter verarbeitet werden soll sowie die genannten Nebenkomponenten und 1,3-Butadien selbst in den Fällen, in denen 1-Buten oder 2-Buten (in der cis- und/oder trans-Form) die gewünschten Wertprodukte sind. Bei Kohlenwasserstoffströmen, die einem FCC-Cracker oder einem Reformer statt einem Steamcracker entstammen, treten analoge Probleme auf.

An Verfahren zur Abreicherung von Alkinen, Dienen, Alkeninen und/oder Polyenen in einem Olefin enthaltenden Kohlenwasserstoffstrom werden beträchtliche Anforderungen gestellt. So stört beispielsweise das im C₂-Strom eines Steamcrackers enthaltene Acetylen die Polymerisation des Ethylens, so dass der Acetylengehalt im C₂-Strom, der typischerweise 0,3 bis 0,8 Vol.-% beträgt, auf Werte unter 1 Vol.-ppm abgesenkt werden muss. Aus dem C₃-Strom eines Steamcrackers müssen Propin und Propadien, die typischerweise in einer Menge von jeweils 2 bis 3 Vol.-% darin enthalten sind, üblicherweise bis auf einen Restgehalt von höchstens 20 Vol.-ppm für Chemieanwendungen oder höchstens 5 Vol.-ppm für Polymeranwendungen entfernt werden. Für den C₄-Kohlenwasserstoffstrom eines Steamcrackers stellt sich die Aufgabe mit Bezug auf C₄-Alkine oder C₄-Alkenine ähnlich dar wie für einen C₃-Strom, wenn 1,3-Butadien aus dem Kohlenwasserstoffstrom als Wertprodukt extrahiert werden soll, oder in einem Kohlenwasserstoffstrom, der bereits von 1,3-Butadien befreit ist, auch mit Bezug auf dessen für die Weiterverarbeitung höchstzulässigen Restgehalt an 1,3-Butadien. Sofern jedoch 1- oder 2-Buten das gewünschte Wertprodukt sind, muss neben den Alkinen, Alkinenen und sonstigen Di- und Polyenen auch 1,3-Butadien, das typischerweise in einer Menge von 30 bis 50 Vol.-% im C₄-Strom enthalten ist, bis auf einen Restgehalt von höchstens 10 Vol.-ppm abgereichert werden.

Üblicherweise werden Alkine, Diene, Alkenine und/oder Polyene aus einem Olefin enthaltenden Kohlenwasserstoffstrom durch selektive katalytische Hydrierung entfernt. C₂-Ströme werden dazu in aller Regel einer Gasphasenhydrierung unterworfen, C₅- und höhere Kohlenwasserstoffströme in aller Regel einer Flüssigphasenhydrierung, für C₃- und C₄-Ströme sind sowohl Gasphasen- als auch Flüssigphasenverfahren bekannt. Als Katalysatoren verwendet man üblicherweise edelmetallhaltige Trägerkatalysatoren, wobei heutzutage meist Palladiumkatalysatoren oder silberdotierte Palladiumkatalysatoren verwendet werden. Je nach dem Gehalt des zu behandelnden Kohlenwasserstoffstroms an den zu entfernenden Verbindungen und ihrer höchstzulässigen Konzentration wird die Hydrierung in nur einem oder - häufiger - in mehreren hintereinander geschalteten Reaktoren durchgeführt. Im letzteren Fall werden meist 2 oder 3 Reaktoren verwendet, wobei im ersten Reaktor ein Umsatzgrad von meist 60 bis 70 % eingestellt wird, im zweiten ein Umsatzgrad von 30 bis 40 % und im letzten Reaktor, sofern vorhanden, der Restumsatz bis in den unteren ppm-Bereich eingestellt wird. Die Verwendung von 4 oder mehr Reaktoren ist zwar möglich, aber meist aus wirtschaftlichen Gründen nicht vorteilhaft. Einen Überblick über die Aufarbeitung von Kohlenwasserstoffströmen aus einem Steamcracker gibt beispielsweise A. Watson in The Oil and Gas Journal, 8. Nov. 1976, S. 179-182.

Bei derartigen Hydrierverfahren sind zwei Bedingungen zu beachten. Einerseits ist auch das gewünschte Wertprodukt wie Ethylen, Propylen, 1,3-Butadien oder 1- oder 2-Buten jeweils eine ungesättigte Verbindung, die am Katalysator hydriert werden kann, was zu Verlust an Wertprodukt führt und daher zu einer sehr selektiven und sorgfältig kontrollierten Hydrierung der unerwünschten Verbindungen zwingt, um aus der höher ungesättigten Verunreinigung ihr gewünschtes olefinisches Homologe und nicht das Alkan zu bilden, oder wenigstens netto kein Wertprodukt durch Hydrierung zum Alkan zu verlieren. Andererseits bilden die unerwünschten Alkine, Di- und Polyene am Katalysator sogenanntes Grünöl, eine Mischung verschiedener Oligomerer und Polymerer, das sich am Katalysator, im Reaktor und in nachgeschalteten Anlagenteilen ablagert und so die Katalysatorstandzeit und die Wartungsintervalle verkürzt, so dass eine sehr schnelle und vollständige Hydrierung dieser unerwünschten Grünöl-bildenden Komponenten gefordert ist.

Es sind verschiedene Verfahren zur selektiven katalytischen Gasphasenhydrierung von Alkinen, Dienen, Alkeninen und/oder Polyenen in einem Olefin enthaltenden Kohlenwasserstoffstrom bekannt. So lehrt DE-A-28 54 698 ein mehrstufiges Hydrierverfahren, bei dem die gesamte Wasserstoffmenge zu Beginn in die erste Reaktionszone eingeführt wird und der zu behandelnde Kohlenwasserstoffstrom aufgeteilt wird und die einzelnen Teilströme jeweils vor den einzelnen Reaktionszonen eingespeist werden. Bei diesem Verfahren wird also jeweils mit einem erheblichen Wasserstoffüberschuss, bezogen auf den Anteil an zu hydrierender Verunreinigung gearbeitet, was die Grünölbildung weitgehend vermeidet, aber zu vergleichsweise hohen Verlusten an Wertprodukt führt. Dieses Verfahren ist daher unüblich.

EP-A-87 980 offenbart das weitaus üblichere Verfahren, nämlich die mehrstufige Hydrierung eines Kohlenwasserstoffstroms, wobei zwischen den einzelnen Reaktionszonen jeweils Wasserstoff zugeführt wird. Auf diese Weise steht in jeder Reaktionszone genau die Wasserstoffmenge zur Verfügung die für die Hydrierung der unerwünschten Verbindungen benötigt wird. Damit wird üblicherweise versucht, die Grünölbildung in ausreichendem Maße zu verhindern, aber gleichzeitig den Ausbeuteverlust durch Hydrierung von Wertprodukt oder Überhydrierung der unerwünschten Verbindung gering zu halten. Watson, *1.c*., lehrt eine Variante dieses Verfahrens, nämlich die Verwendung einer Wasserstoffmenge, die zu einem minimalen Wasserstoffüberschuss am Ausgang der einzelnen Reaktoren führt. Weitere Maßnahmen zur Optimierung derartiger Hydrierverfahren sind beispielsweise eine sorgfältige Temperaturkontrolle, wie in US-A-4,707,245 offenbart. Eine altbekannte Methode zur Selektivitätserhöhung des Katalysators in derartigen Verfahren ist die Zugabe von Kohlenmonoxid als Moderator, wodurch der Katalysator selektiver, aber inaktiver wird; dies hat allerdings die Nachteile, dass dieses Kohlenmonoxid wieder abgetrennt werden muss und dass die niedrigere Aktivität des Katalysators durch höhere Betriebstemperatur ausgeglichen werden muss, was die Grünölbildung begünstigt.

Die Forderung nach möglichst niedrigen Wertproduktverlust erzwingt auch die Entwicklung und Verwendung hochselektiver Katalysatoren, wie sie beispielsweise in EP-A-992 284 und der darin zitierten Literatur beschrieben sind. Es ist auch bekannt statt der verbreiteten stückigen Katalysatoren strukturierte Katalysatoren, Monolithe oder Katalysatorpackungen einzusetzen, wie sie beispielsweise in US-A-5,866,734 oder in EP-A-965 384 offenbart werden.

Angesichts der immer höheren Anforderungen an die Reinheit von Olefinen, dem Wunsch nach möglichst geringem Wertproduktverlust und dem Wunsch nach langen Standzeiten von Katalysatoren und langen Wartungsintervallen von Anlagen stellt sich die Aufgabe, ein Verfahren zur Entfernung von Alkinen, Di- und/oder Polyenen aus Olefin enthaltenden Kohlenwasserstoffströmen zu finden, das einerseits die Bildung von Grünöl wirksam verhindert und andererseits mit hoher Selektivität zum gewünschten Wertprodukt führt. Dementsprechend wurde ein Verfahren zur selektiven katalytischen Gasphasenhydrierung von Alkinen, Dienen, Alkeninen und/oder Polyenen in einem Olefin enthaltenden Kohlenwasserstoffstrom in mindestens zwei hintereinandergeschalteten Reaktionszonen ohne Zuführung eines Teils dieses Kohlenwasserstoffstroms zwischen der vorletzten und der letzten Reaktionszone gefunden, das dadurch gekennzeichnet ist, dass man den Wasserstoffgehalt im Reaktionsgasgemisch vor der vorletzten Reaktionszone und den Umsatzgrad in der vorletzten Reaktionszone so einstellt, dass das Reaktionsgasgemisch am Ausgang der vorletzten Reaktionszone mindestens 0,7 Vol.-% Wasserstoff enthält.

Mit dem erfindungsgemäßen Verfahren wird die Grünölbildung weitgehend zurückgedrängt, während gleichzeitig der Verlust an Wertprodukt minimiert wird oder kein derartiger Verlust auftritt. Überraschenderweise wird mit dem erfindungsgemäßen Verfahren trotz des angewendeten vergleichsweise hohen Wasserstoffüberschusses kein Anstieg der Überhydrierung zu Alkanen beobachtet.

Mit dem erfindungsgemäßen Verfahren werden Alkine, Diene, Alkenine und/oder Polyene in einem Olefin enthaltenden Kohlenwasserstoffstrom selektiv katalytisch und in der Gasphase hydriert. Insbesondere wird mit dem erfindungsgemäßen Verfahren Acetylen in einem Ethylen enthaltenden C₂-Strom hydriert, Propin und Propadien in einem Propylen enthaltenden C₃-Strom oder 1-Butin, 2-Butin, But-3-en-1-in, 1,2-Butadien, Butatrien und/oder 1,3-Butadien in einem 1,3-Butadien und/oder 1-Buten oder 2-Buten enthaltenden Kohlenwasserstoffstrom.

Das erfindungsgemäße Verfahren wird in mindestens zwei hintereinandergeschalteten Reaktionszonen durchgeführt, vorzugsweise in zwei oder drei hintereinandergeschalteten Reaktionszonen. Es ist ebenso möglich, vier oder mehr hintereinandergeschaltete Reaktionszonen zu verwenden, diese Ausführungsform ist jedoch aus wirtschaftlichen Gründen meist nicht vorteilhaft, es sei denn, dass die zu entfernenden Alkine, Diene, Alkenine und/oder Polyene in ungewöhnlich hoher Menge im Kohlenwasserstoffstrom enthalten sind. Unter "Reaktionszone" ist im Rahmen der vorliegenden Erfindung ein einzelner Reaktor zu verstehen oder ein einzelner Abschnitt eines Reaktors, in dem mehrere Reaktionszonen (beispielsweise einzelne, räumlich voneinander getrennte Katalysatorbetten) in einem gemeinsamen Reaktormantel untergebracht sind. Sofern Reaktionszonen adiabat betrieben werden, sind im Anschluss an die adiabat betriebene Reaktionszone Kühlvorrichtungen angeordnet, um die entstehende Reaktionswärme zumindest teilweise aus dem Produktstrom abzuführen, oder es werden andere bekannte Kilhlmaßnahmen getroffen, beispielsweise die Rückführung eines Teils des Produkts einer Reaktionszone an den Anfang dieser Reaktionszone, nachdem dieser sogenannte Kreisgasstrom gekühlt wurde. Alternativ können die einzelnen Reaktionszonen auch isotherm, also mit Kühlvorrichtungen im Katalysatorbett selbst betrieben werden. Die Kühlvorrichtungen werden der bei der selektiven Hydrierung des gegebenen Kohlenwasserstoffstroms entstehenden Wärmemenge angepasst und können, falls diese Wärmemenge entsprechend gering ist oder ein entsprechend heißer Produktstrom gewünscht wird, auch entfallen; dies ist Bestandteil einer üblichen Reaktor- und Verfahrensauslegung.

Ferner sind mindestens vor der ersten und vor der vorletzten Reaktionszone Einrichtungen zum Einleiten von Wasserstoff in das Reaktionsgasgemisch angeordnet. Vorzugsweise ist vor jeder Reaktionszone eine Einrichtung zum Einleiten von Wasserstoff in das Reaktionsgasgemisch angeordnet.

Der zu behandelnde Kohlenwasserstoffstrom durchläuft die einzelnen Reaktionszonen im Regelfall hintereinander. Es ist zwar möglich, aber nicht erforderlich, diesen Kohlenwasserstoffstrom in einzelne Teilströme aufzutrennen und diese Teilströme - abgesehen von dem in die erste Reaktionszone geführten Teilstrom - jeweils zwischen zwei Reaktionszonen einzuleiten. Insbesondere ist die Einleitung einer Teilmenge des zu behandelnden Kohlenwasserstoffstroms zwischen der vorletzten und der letzten Reaktionszone nicht notwendig. Es ist jedoch möglich, verschiedene Kohlenwasserstoffströme entsprechend ihrem jeweiligen Gehalt an Alkinen, Dienen, Alkeninen und/oder Polyenen zwischen den einzelnen Reaktionszonen einzuleiten. Sind in einem gegebenen

Anlagenkomplex beispielsweise ein erster Kohlenwasserstoffstrom mit relativ hohem Anteil an Alkinen, Dienen, Alkeninen und/oder Polyenen und gleichzeitig ein ansonsten vergleichbarer zweiter Kohlenwasserstoffstrom mit niedrigerem Gehalt dieser Verbindungen selektiv zu hydrieren, so wird der erste Strom erfindungsgemäß in mehreren Reaktionszonen hydriert, und der zweite Strom wird zwischen zwei Reaktionszonen eingeleitet, an einer Stelle, an der der ursprünglich höhere Gehalt des ersten Kohlenwasserstoffstroms an den zu hydrierenden Verbindungen bereits entsprechend erniedrigt ist.

Ein Teilstrom des Produkts einer Reaktionszone kann aus dem Produktgasstrom entnoammen und vor dieser Reaktionszone dem zu hydrierenden Gasstrom wieder zugeführt werden (im Prinzip ist auch die Zuführung vor einer anderen Reaktionszone möglich). Diese sogenannte "Kreisgasfahrweise" ist eine bei derartigen Hydrierungen häufig angewendete Maßnahme und dient insbesondere der Einstellung eines ausreichenden Umsatzes in einer bestimmten Reaktionszone, wobei das Kreisgas vor seiner Rückführung auch gekühlt werden kann, so dass der gewünschte Umsatz erreicht wird, ohne dass das Produkt durch die freiwerdende Reaktionswärme unerwünscht heiß wird. Typische Rücklaufverhältnisse (rückgeführter Teilstrom zu erstmals in die betreffende Reaktionszone eingeführter Teilstrom) liegen im Bereich von 0 bis 30.

Die in den einzelnen Reaktionszonen eingestellten Bedingungen entsprechen mit Ausnahme des erfindungsgemäß einzustellenden Wasserstoffüberschusses am Ausgang der vorletzten Reaktionszone üblichen Bedingungen für derartige selektive Hydrierungen und werden gemäß den anlagenspezifischen Rahmenbedingungen und dem zu erreichenden Reinheitsgrad eingestellt. Zur selektiven Hydrierung von Acetylen in C₂-Strömen wird üblicherweise eine Raumgeschwindigkeit des gasförmigen C₂-Stroms von 500 m³/m³*h, bezogen auf das Katalysatorvolumen, bis 10 m³/m³*h bei einer Temperatur von 0°C bis 250°C und einem Druck von 0,01 bar bis 50 bar (jeweils Überdruck, barü, englisch: "gauge", barg) eingestellt und je Mol Acetylen im C₂-Strom insgesamt (d.h., über alle Reaktionszonen) 1 bis 2 Mol Wasserstoff zugegeben. Zur selektiven Hydrierung von Propin und Propadien in C₃-Strömen wird üblicherweise eine Raumgeschwindigkeit des gasförmigen C₃-Stroms von 500 m³/m³*h, bezogen auf das Katalysatorvolumen, bis 10000 m³/m³*h bei einer Temperatur von 0°C bis 250°C und einem Druck von 1 bar bis 50 bar eingestellt und je Mol Propin und Propadien im C₃-Strom insgesamt 1 bis 3 Mol Wasserstoff zugegeben. Zur selektiven Hydrierung von Alkinen, Dienen, Alkeninen und/oder Polyenen in C₄-Strömen wird üblicherweise eine Raumgeschwindigkeit des gasförmigen C₄-Stroms von 200 m³/m³*h, bezogen auf das Katalysatorvolumen, bis 10000 m³/m³*h bei einer Temperatur von 0°C bis 300°C und einem Druck von 1 bar bis 30 bar eingestellt und je Mol zu hydrierender Kohlenstoff-Kohlenstoff-Mehrfachbindung in den zu entfernenden Alkinen, Dienen, Alkeninen und/oder Polyenen 1 bis 10 Mol Wasserstoff zugegeben.

Der Wasserstoff kann vollständig vor der ersten Reaktionszone zugesetzt werden. Bevorzugterweise wird der Wasserstoff jedoch zwischen den einzelnen Reaktionszonen zugeführt, und zwar jeweils in Teilmengen, die so bemessen sind, dass in der darauffolgenden Reaktionszone zwar der in dieser Stufe gewünschte Umsatzgrad der zu hydrierenden Alkine, Diene, Alkenine und/oder Polyene erreicht wird, aber die unerwünschte Überhydrierung von Wertprodukten zu Alkanen ausbleibt oder zumindest nur in tolerierbar geringem Umfang anfällt.

Der erforderliche Gesamtumsatz an Alkinen, Dienen, Alkeninen und/oder Polyenen über alle Reaktionsstufen ist durch die tolerierbaren Restmengen an diesen Verbindungen im selektiv hydrierten Produktstrom, die wiederum durch dessen weitere Verwendung bestimmt werden und typischerweise im Bereich weniger Vol.-ppm liegen, vorgegeben. Ein Gesamtumsatz von exakt 100 % (d.h. ein Restgehalt an Alkinen, Dienen, Alkeninen und/oder Polyenen von exakt 0 Vol.-ppm) wird zumeist nicht eingestellt, da dabei ein unerwünscht hoher Verlust an Wertprodukt durch Hydrierung zum entsprechenden Alkan auftreten würde. Üblicherweise wird die erste Reaktionszone so betrieben, dass der überwiegende Teil des Gesamtumsatzes dort erfolgt, ein typischer Wert sind 60 bis 70 Mol-% Umsatz, bezogen auf die ursprünglich vorhandenen Alkine, Diene, Alkenine und/oder Polyene. Werden nur zwei Reaktionsstufen verwendet, so wird in der ersten zumeist ein tendenziell etwas höherer Umsatzgrad eingestellt als im Falle eines drei- oder mehrstufigen Verfahrens. Bei einer zweistufigen Verfahrensdurchführung wird in der zweiten Reaktionszone der Restumsatz zur Erreichung des gewünschten maximalen Restgehalts an Alkinen, Dienen, Alkeninen und/oder Polyenen eingestellt. Wird das Verfahren dreistufig durchgeführt, so beträgt ein typischer Umsatzgrad in der zweiten Reaktionszone 30 bis 40 Mol-%, so dass am Ausgang der zweiten Reaktionszone ein Gesamtumsatz von mehr als 90 Mol-% und bis zu annähernd 100 Mol-% erreicht wird. Im dritten Reaktor wird dann der gewünschte Restumsatz eingestellt, der zur Entfernung der Alkine, Diene, Alkenine und/oder Polyene bis auf den tolerierbaren Restgehalt erforderlich ist. Werden mehr als drei Reaktionszonen verwendet, so wird der Umsatz analog auf die verwendeten Reaktionszonen aufgeteilt. Die Einstellung des Umsatzes erfolgt wie üblich durch entsprechende Einstellung der Verfahrensparameter wie Temperatur, Raumgeschwindigkeit, Druck oder Rücklaufverhältnis.

Der Gehalt des selektiv zu hydrierenden Einsatzstoffstroms an zu entfernenden Alkinen, Dienen, Alkeninen und/oder Polyenen bestimmt, wieviele Reaktionszonen im Einzelfall zu verwenden sind. Beispielsweise wird - bei typischen Alkin-, Dien und/oder Polyen-Gehalten - ein C₂-Kohlenwasserstoffstrom in zwei und ein C₃-Kohlenwasserstoffstrom in zwei oder drei Reaktionszonen behandelt. Die sonstigen verfahrenstechnischen Gegebenheiten können auch Einfluss auf die Reaktionszonenzahl haben, beispielsweise kann eine isotherm betriebene Reaktionszone zwei oder mehr adiabat betriebene Reaktionszonen, zwischen denen eine Zwischenkühlung zur Abführung der Reaktionswärme anzuordnen wäre, ersetzen.

Im erfindungsgemäßen Verfahren wird der Wasserstoffgehalt vor der vorletzten Reaktionszone und der Umsatz in der vorletzten Reaktionszone so eingestellt, dass das Reaktionsgemisch am Ausgang der vorletzten Reaktionszone mindestens 0,7 Vol.-% Wasserstoff enthält. Vorzugsweise beträgt dieser Wasserstoffgehalt mindestens 0,8 Vol.-% und in besonders bevorzugter Weise mindestens 0,9 Vol.-%. Er beträgt ferner im Allgemeinen höchstens 2 Vol.-%, vorzugsweise höchstens 1,8 Vol.-% und in besonders bevorzugter Weise höchstens 1,6 Vol.-%. Maßnahmen zur Einstellung eines bestimmten Wasserstoffgehalts am Ausgang eines Reaktors oder einer Reaktionszone sind bekannt. Insbesondere kann die Temperatur in dieser Reaktionszone soweit abgesenkt werden, etwa durch entsprechende Kühlung des Reaktors bei isothermer Betriebsweise oder des Reaktorzulaufs bei adiabater Betriebsweise und/oder der Durchsatz durch den Reaktor kann soweit erhöht werden, dass der Wasserstoffumsatz in dieser Reaktionszone nicht 100 Mol-% beträgt, sondern soweit absinkt, dass der gewünschte Wasserstoffgehalt am Ausgang dieser Reaktionszone erhalten wird. Ferner kann der Wasserstoffüberschuss vor dieser Reaktionszone so hoch gewählt werden (durch Verringerung des Wasserstoffumsatzes in einer oder mehreren vorher durchlaufenen Reaktionszonen oder durch entsprechende Wasserstoffdosierung vor der vorletzten Reaktionszone), dass der gewünschte Wasserstoffgehalt am Ausgang der vorletzten Reaktionszone eingestellt wird.

Die sonstige Auslegung der Anlage zur Durchführung des erfindungsgemäßen Verfahrens, einschließlich der Festlegung der verwendeten Reaktionszonenanzahl, der Wärmeabführung, von etwaigen Rückführungen, und die Wahl der sonst im Betrieb angewendeten Temperaturen, Drücke, Raumgeschwindigkeiten und anderen verfahrenstechnischen Parameter erfolgt wie bei derartigen Hydrierverfahren allgemein üblich.

Als Katalysatoren in den einzelnen Reaktionszonen verwendet man im Allgemeinen Katalysatoren, die zur Hydrierung von Alkinen, Dienen, Alkeninen und/oder Polyenen in Olefin enthaltenden Kohlenwasserstoffströmen geeignet sind. Die Verwendung hochselektiver Katalysatoren (also solcher, die bevorzugt Alkine, Diene, Alkenine und/oder Polyene zu Olefinen hydrieren und nur in geringem Umfang Olefine zu Alkanen) ist bevorzugt. Bei weniger selektiven Katalysatoren kann die Einstellung des erfindungsgemäßen Wasserstoffgehalts sogar unmöglich sein, nämlich dann, wenn in Gegenwart dieser Katalysatoren jeglicher vorhandene Wasserstoff mit den bei derartigen Verfahren naturgemäß im Überschuss vorliegenden Olefinen vollständig zu Alkanen umgesetzt wird. Im allgemeinen ist für das erfindungsgemäße Verfahren daher jeder Katalysator zur Hydrierung von Alkinen, Dienen, Alkeninen und/oder Polyenen verwendbar, der so selektiv ist, dass er die Einstellung des erfindungsgemäßen Wasserstoffgehalts erlaubt. Dies kann, falls nötig, in einem Routineversuch festgestellt werden.

Bekannte hochselektive Katalysatoren zur Hydrierung von Alkinen, Dienen, Alkeninen und/oder Polyenen in Olefin enthaltenden Kohlenwasserstoffströmen, die auch im erfindungsgemäßen Verfahren verwendet werden können, enthalten typischerweise ein Metall der Gruppe 10 des Periodensystems der Elemente (Nickel, Palladium, Platin) und wahlweise auch ein Metall der Gruppe 11 des Periodensystems der Elemente (Kupfer, Silber, Gold) auf einem Katalysatorträger. Oft werden Palladiumkatalysatoren oder silberdotierte Palladiumkatalysatoren auf einem stückigen (partikelförmigen) oxidischen Träger, häufig Aluminiumoxid, verwendet. Derartige Katalysatoren und ihre Herstellung sind wohlbekannt, siehe zum Beispiel die eingangs genannte EP-A-992 284 und die darin zitierten Schriften, auf die hiermit ausdrücklich Bezug genommen wird.

Im Allgemeinen wird in mindestens einer Reaktionszone ein Katalysator verwendet, der ein Metall der Gruppe 10 des Periodensystems der Elemente auf einem Katalysatorträger enthält. Wahlweise enthält dieser Katalysator auch ein Element der Gruppe 11 des Periodensystems der Elemente. In bevorzugter Weise enthält der Katalysator als Metall der 10. Gruppe des Periodensystems der Elemente Palladium und als Metall der 11. Gruppe des Periodensystems der Elemente Silber. In ebenso bevorzugter Weise ist der Katalysatorträger ein oxidischer Katalysatorträger, beispielsweise Aluminiumoxid. Insbesondere wird ein derartiger Katalysator in der vorletzten Reaktionszone verwendet.

In besonders bevorzugter Weise setzt man beim erfindungsgemäßen Verfahren zumindest in der vorletzten Reaktionszone einen Katalysator ein, der ein Metall der Gruppe 10 des Periodensystems der Elemente (Nickel, Palladium, Platin) und wahlweise auch ein Metall der Gruppe 11 (Kupfer, Silber, Gold) des Periodensystems der Elemente auf einem strukturierten, aus Drahtgewebe, -gestrick, -gewirk, -filz oder Folien oder Blechen, die wahlweise auch perforiert sind, aufgebauten Katalysatorträger oder Monolith enthält. Vorzugsweise enthält der Katalysator Palladium und wahlweise auch Silber. Derartige Katalysatoren und ihre Herstellung sind ebenfalls wohlbekannt, siehe zum Beispiel die eingangs genannten US-A-5,866,734, EP-A-827,944 und EP-A-965,384 und die jeweils darin zitierten Schriften, auf die hiermit ausdrücklich Bezug genommen wird.

### Beispiel

Der zweite Reaktor einer üblichen Anlage, bestehend aus drei adiabat betriebenen hintereinandergeschalteten Reaktoren, zur Hydrierung von Alkinen, Dienen, Alkeninen und/oder Polyenen in einem Propen enthaltenden C₃-Strom eines Steamcrackers, wurde mit einem Palladium/Silber-Katalysator auf einem Drahtgestrickträger in Monolithform (hergestellt gemäß EP-A-965 384) ausgerüstet. Die Anlage wurde bestimmungsgemäß mit C₃-Strom mit konventionellen Parametern betrieben. Nach einer Laufzeit von 90 Tagen wurde die Temperatur des zweiten Reaktors vom üblichen Wert im Bereich von ca. 80 bis 85°C auf einen Wert im Bereich von etwa 65°C abgesenkt, wodurch sich der Wasserstoffgehalt im Reaktionsgemisch am Ausgang des zweiten Reaktors auf Werte im Bereich von 0,8 bis 1,8 Vol.-% erhöhte. Die anderen beiden Stufen wurden dabei weiterhin mit konventionellen Katalysatoren und üblichen Betriebsbedingungen betrieben.

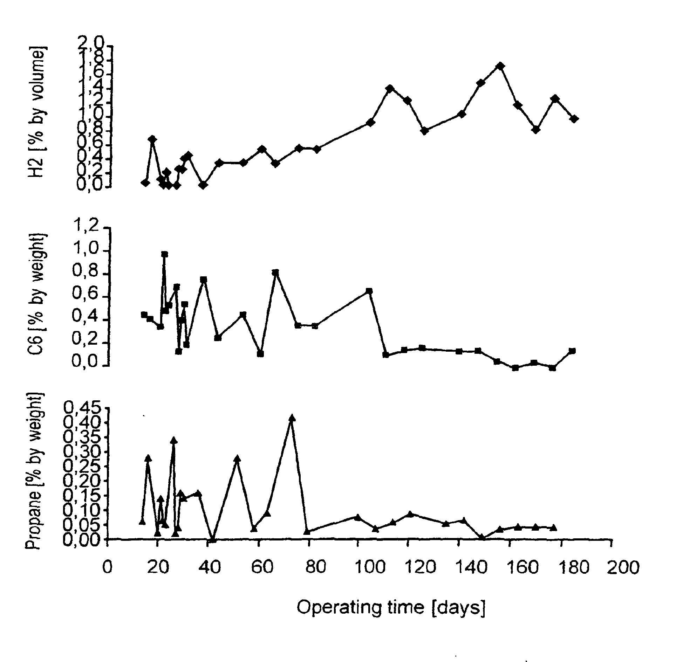

Abbildung 1 stellt die Ergebnisse im Einzelnen grafisch dar.

Überraschenderweise zeigte sich, dass trotz dieses Überangebots an Wasserstoff (H2) die unerwünschte Überhydrierung zu Propan, d.h., der Propangehalt des Reaktoraustrags, tendenziell eher abnahm und sich auf niedrigen Werten stabilisierte, und die Grünölbildung, ausgedrückt als Gehalt des Reaktoraustrags an C₆-Verbindungen, deutlich zurückging.

## Patentansprüche

1. Verfahren zur selektiven katalytischen Gasphasenhydrierung von Alkinen, Dienen, Alkeninen und/oder Polyenen in einem Olefin enthaltenden Kohlenwasserstoffstrom in mindestens zwei hintereinandergeschalteten Reaktionszonen ohne.Zuführung eines Teils dieses Kohlenwasserstoffstroms zwischen der vorletzten und der letzten Reaktionszone, **dadurch gekennzeichnet, dass** man den Wasserstoffgehalt im Reaktionsgemisch vor der vorletzten Reaktionszone und den Umsatzgrad in der vorletzten Reaktionszone so einstellt, dass das Reaktionsgemisch am Ausgang der vorletzten Reaktionszone mindestens 0,7 Vol.-% Wasserstoff enthält.

2. Verfahren nach Anspruch 1, **dadurch gekennzeichnet, dass** man Acetylen in einem C2-Strom hydriert.

3. Verfahren nach Anspruch 1, **dadurch gekennzeichnet, dass** man Propin und Propadien in einem C3-Strom hydriert.

4. Verfahren nach Anspruch 1, **dadurch gekennzeichnet, dass** man Butin, But-3-en-1-in, 1,2-Butadien und/oder 1,3-Butadien in einem C4-Strom hydriert.

5. Verfahren nach Anspruch 1, **dadurch gekennzeichnet, dass** man Alkine, Diene und/oder Polyene in einem Olefin enthaltenden Kohlenwasserstoffstrom in zwei oder drei hintereinandergeschalteten Reaktionszonen hydriert.

6. Verfahren nach Anspruch 1, **dadurch gekennzeichnet, dass** man in den Reaktionszonen Katalysatoren verwendet, die ein Metall der Gruppe 10 des Periodensystems der Elemente auf einem Katalysatorträger enthalten.

7. Verfahren nach Anspruch 6, **dadurch gekennzeichnet, dass** man in mindestens einer Reaktionszone einen Katalysator verwendet, der ein Metall der Gruppe 10 und ein Metall der Gruppe 11 auf einem Katalysatorträger enthält.

8. Verfahren nach Anspruch 6, **dadurch gekennzeichnet, dass** man Katalysatoren verwendet, die Palladium auf einem Katalysatorträger enthalten.

9. Verfahren nach Anspruch 7, **dadurch gekennzeichnet, dass** man in mindestens einer Reaktionszone einen Katalysator verwendet, der Palladium und Silber auf einem Katalysatorträger enthält.

10. Verfahren nach Anspruch 6, **dadurch gekennzeichnet, dass** man in der vorletzten Reaktionszone einen Katalysator verwendet, der ein Metall der Gruppe 10 und wahlweise auch ein Metall der Gruppe 11 auf einem strukturierten, aus Drahtgewebe, -gestrick, -gewirk, -filz oder Folien oder Blechen, die wahlweise auch perforiert sind, aufgebauten Katalysatorträger oder Monolith enthält.

## Claims

1. A process for the selective catalytic gas-phase hydrogenation of alkynes, dienes, alkenynes and/or polyenes in an olefin-comprising hydrocarbon stream in at least two reaction zones connected in series without introduction of part of this hydrocarbon stream between the penultimate reaction zone and the last reaction zone, wherein the hydrogen content in the reaction mixture upstream of the penultimate reaction zone and the degree of conversion in the penultimate reaction zone are set so that the reaction mixture comprises at least 0.7% by volume of hydrogen at the outlet of the penultimate reaction zone.

2. The process according to claim 1, wherein acetylene in a C2 stream is hydrogenated.

3. The process according to claim 1, wherein propyne and propadiene in a C3 stream are hydrogenated.

4. The process according to claim 1, wherein butyne, but-3-en-1-yne, 1,2-butadiene and/or 1,3-butadiene in a C4 stream are hydrogenated.

5. The process according to claim 1, wherein alkynes, dienes and/or polyenes in an olefin-comprising hydrocarbon stream are hydrogenated in two or three reaction zones connected in series.

6. The process according to claim 1, wherein catalysts comprising a metal of group 10 of the Periodic Table of the Elements on a catalyst support are used in the reaction zones.

7. The process according to claim 6, wherein a catalyst comprising a metal of group 10 and a metal of group 11 on a catalyst support is used in at least one reaction zone.

8. The process according to claim 6, wherein catalysts comprising palladium on a catalyst support are used.

9. The process according to claim 7, wherein a catalyst comprising palladium and silver on a catalyst support is used in at least one reaction zone.

10. The process according to claim 6, wherein a catalyst comprising a metal of group 10 and optionally a metal of group 11 on a structured catalyst support or monolith made up of woven wire mesh, knitted wire mesh, wire felt or foils or metal sheets, which may if desired be perforated.

## Revendications

1. Procédé d'hydrogénation catalytique sélective en phase gazeuse d'alcynes, de diènes, d'alcénynes et/ou de polyènes dans un courant d'hydrocarbure contenant de l'oléfine dans au moins deux zones réactionnelles montées successivement, sans apport d'une partie de ce courant d'hydrocarbure entre l'avant-dernière zone réactionnelle et la dernière, **caractérisé en ce qu'**on ajuste la teneur en hydrogène dans le mélange réactionnel avant l'avant-dernière zone réactionnelle et le degré de conversion dans l'avant-dernière zone réactionnelle de façon à ce que le mélange réactionnel à la sortie de l'avant-dernière zone réactionnelle contienne au moins 0,7 % en volume d'hydrogène.

2. Procédé suivant la revendication 1, **caractérisé en ce qu'**on hydrogène de l'acétylène dans un courant en C2.

3. Procédé suivant la revendication 1, **caractérisé en ce qu'**on hydrogène du propyne et du propadiène dans un courant en C3.

4. Procédé suivant la revendication 1, **caractérisé en ce qu'**on hydrogène du butyne, du but-3-én-1-yne, du 1,2-butadiène et/ou du 1,3-butadiène dans un courant en C4.

5. Procédé suivant la revendication 1, **caractérisé en ce qu'**on hydrogène des alcynes, des diènes et/ou des polyènes dans un courant d'hydrocarbure contenant de l'oléfine dans deux ou trois zones réactionnelles montées successivement.

6. Procédé suivant la revendication 1, **caractérisé en ce que**, dans les zones réactionnelles, on utilise des catalyseurs qui contiennent un métal du groupe 10 du système périodique des éléments sur un support de catalyseur.

7. Procédé suivant la revendication 6, **caractérisé en ce que**, dans au moins une zone réactionnelle, on utilise un catalyseur qui contient un métal du groupe 10 et un métal du groupe 11 sur un support de catalyseur.

8. Procédé suivant la revendication 6, **caractérisé en ce qu'**on utilise des catalyseurs qui contiennent du palladium sur un support de catalyseur.

9. Procédé suivant la revendication 7, **caractérisé en ce que**, dans au moins une zone réactionnelle, on utilise un catalyseur qui contient du palladium et de l'argent sur un support de catalyseur.

10. Procédé suivant la revendication 6, **caractérisé en ce que**, dans l'avant-dernière zone réactionnelle, on utilise un catalyseur qui contient un métal du groupe 10 et au choix également un métal du groupe 11 sur un monolithe ou support de catalyseur structuré, constitué de tissu, tricot, maillage ou feutre métalliques ou de feuilles ou tôles qui sont au choix également perforées.
